# EUROPÄISCHE PATENTANMELDUNG

(11) **EP 3 061 442 A1**
(43) Veröffentlichungstag der Anmeldung: **31.08.2016**
(21) Anmeldenummer: 15156961.3
(22) Anmeldetag: 27.02.2015
(51) Int. Cl.: A61K 8/60, A61Q 19/10, A61K 8/891, A61K 8/894

(54) **Zusammensetzung enthaltend Rhamnolipid und Siloxan**

(71) Anmelder: Evonik Degussa GmbH, 45128 Essen (DE)
(72) Erfinder: Scheuermann, Ralph, 45359 Essen (DE); Pickl, Marcel, 46236 Bottrop (DE); Ferenz, Michael, 45147 Essen (DE); Wenk, Hans Henning, 45470 Mülheim an der Ruhr (DE); Schilling, Martin, 53121 Bonn (DE); Brandt, Kathrin Daniela, 40476 Düsseldorf (DE); Dahl, Verena, 51515 Kürten (DE)

(57) **Zusammenfassung**

Gegenstand der Erfindung sind Zusammensetzungen enthaltend mindestens ein Rhamnolipid und mindestens ein Siloxan, dadurch gekennzeichnet, dass das Gewichtsverhältnis von Rhamnolipid zu Siloxan von 5.000.000 zu 1 bis 100 zu 1, bevorzugt von 500.000 zu 1 bis 1.000 zu 1, besonders bevorzugt von 25.000 zu 1 bis 2.500 zu 1 beträgt.

## Beschreibung

### Gebiet der Erfindung

Gegenstand der Erfindung sind Zusammensetzungen enthaltend mindestens ein Rhamnolipid und mindestens ein Siloxan, dadurch gekennzeichnet, dass das Gewichtsverhältnis von Rhamnolipid zu Siloxan von 5.000.000 zu 1 bis 100 zu 1, bevorzugt von 500.000 zu 1 bis 1.000 zu 1, besonders bevorzugt von 25.000 zu 1 bis 2.500 zu 1 beträgt.

### Stand der Technik

Rhamnolipide zur Verwendung in Kosmetik oder als Reinigungsmittel sind seit langem bekannt. So beschreibt beispielsweise die EP2786742 kosmetische Formulierungen enthaltend mindestens ein Rhamnolipid.

Die EP2786743 offenbart Mischungszusammensetzungen enthaltend Rhamnolipide sowie ihre Verwendung zur Herstellung von kosmetischen Formulierungen oder Reinigungsformulierungen. Die EP2787065 offenbart Waschmittelformulierung für Textilien enthaltend Rhamnolipide mit einem überwiegenden Gehalt an di-Rhamnolipiden.

Aufgabe der Erfindung war es, Zusammensetzungen bereitzustellen, die zu einer verbesserten Entfernung unerwünschter Gerüche auf der Haut führen.

### Beschreibung der Erfindung

Überraschenderweise wurde gefunden, dass Zusammensetzungen enthaltend Rhamnolipide die der Erfindung gestellte Aufgabe zu lösen vermögen, wenn nur geringe Mengen an Siloxan enthalten sind.

Gegenstand der vorliegenden Erfindung sind daher Zusammensetzungen enthaltend mindestens ein Rhamnolipid und mindestens ein Siloxan, dadurch gekennzeichnet, dass das Gewichtsverhältnis von Rhamnolipid zu Siloxan von 5.000.000 zu 1 bis 100 zu 1, bevorzugt von 500.000 zu 1 bis 1.000 zu 1, besonders bevorzugt von 25.000 zu 1 bis 2.500 zu 1 beträgt.

Ein weiterer Gegenstand der Erfindung sind Formulierungen enthaltend die erfindungsgemäßen Zusammensetzungen.

Ein weiterer Vorteil der vorliegenden Erfindung ist es, dass die erfindungsgemäßen Zusammensetzungen ein überragendes Reinigungsvermögen gepaart mit dem Erhalt der Struktur von Wildleder aufweisen können.

Noch ein Vorteil der vorliegenden Erfindung ist es, dass die erfindungsgemäßen Zusammensetzungen unerwünschte Gerüche von Haut beseitigen können und gleichzeitig ein gutes Hautgefühl hinterlassen.

Noch ein Vorteil der vorliegenden Erfindung ist es, dass die erfindungsgemäßen Zusammensetzungen in tensidischen Formulierungen eine große Menge Schaum hervorbringen können.

Ein weiterer Vorteil der vorliegenden Erfindung ist es, dass die erfindungsgemäßen Zusammensetzungen in tensidischen Formulierungen einen gut verteilbaren Schaum, insbesondere auf Haut und Haaren, hervorbringen können. Sie eignen sich z.B. für die Herstellung von Rasierschäumen mit verbesserter Konsistenz und Verteilbarkeit auf der Haut.

Ein weiterer Vorteil der vorliegenden Erfindung ist, dass die Schäume eine feinere Porenstruktur sowie einen höheren Wassergehalt und dadurch eine bessere Haptik aufweisen.

Ein weiterer Vorteil der vorliegenden Erfindung ist es, dass die erfindungsgemäßen Zusammensetzungen einen erhöhten Glanz auf harten Oberflächen wie zum Beispiel Kunststoff und Glas bei gleichzeitig hervorragender Reinigungsleistung hervorrufen können.

Noch ein Vorteil der vorliegenden Erfindung ist es, dass die erfindungsgemäßen Zusammensetzungen ein überragendes Reinigungsvermögen gepaart mit einer guten Wiederbenetzbarkeit von Textilien aufweisen können.

Unter dem Begriff "Rhamnolipid" im Zusammenhang mit der vorliegenden Erfindung werden insbesondere Verbindungen der allgemeinen Formel (I) oder deren Salze verstanden, 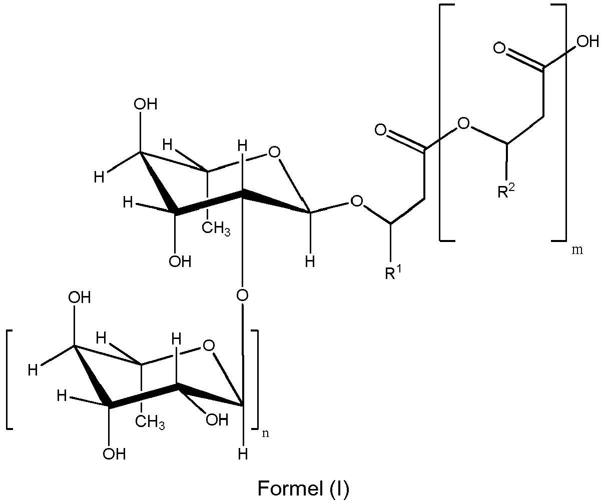 wobei
m = 2, 1 oder 0,
n = 1 oder 0,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH₂)ₒ-CH₃ mit o = 1 bis 23, bevorzugt 4 bis 12.

Unter dem Begriff "di-Rhamnolipid" im Zusammenhang mit der vorliegenden Erfindung werden Verbindungen der allgemeinen Formel (I) oder deren Salze verstanden, bei denen n =1.

Unter dem Begriff "mono-Rhamnolipid" im Zusammenhang mit der vorliegenden Erfindung werden Verbindungen der allgemeinen Formel (I) oder deren Salze verstanden, bei denen n =0.

Distinkte Rhamnolipide werden gemäß folgender Nomenklatur abgekürzt:
Unter "diRL-CXCY" werden di-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.
Unter "monoRL-CXCY" werden mono-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Die verwendete Nomenklatur unterscheidet somit nicht zwischen "CXCY" und "CYCX".

Für Rhamnolipide mit m=0 wird entsprechend monoRL-CX bzw. diRL-CX verwendet.

Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.

Zur Bestimmung des Gehaltes an Rhamnolipiden im Zusammenhang mit der vorliegenden Erfindung wird lediglich die Masse des Rhamnolipid-Anions, somit "allgemeinen Formel (I) abzüglich ein Wasserstoff" berücksichtigt.

Zur Bestimmung des Gehaltes an Rhamnolipiden im Zusammenhang mit der vorliegenden Erfindung werden alle Rhamnolipide durch Ansäuern in die protonierte Form (vgl. allgemeine Formel (I)) überführt und mittels HPLC quantifiziert.

Der Gehalt an Siloxan lässt sich beispielsweise unter Verwendung eines geeigneten internen Standards, bevorzugt dem identischen Siloxan, mittels ¹H-NMR (Kernresonanzspektroskopie), gegebenenfalls mit vorheriger extrakiver oder chromatographischer Anreicherung zur Erhöhung der Nachweisgrenze, bestimmen.

Alle angegebenen Prozent (%) sind, wenn nicht anders angegeben, Massenprozent.

Der Begriff ppm (=parts per million) beschreibt ebenfalls Massenanteile.

Unter dem Begriff "wässrig" im Zusammenhang mit der vorliegenden Erfindung ist eine Zusammensetzung zu verstehen, die mindestens 5 Gew.-% Wasser, bezogen auf die betrachtete Gesamtzusammensetzung enthält.

Der "pH-Wert" im Zusammenhang mit der vorliegenden Erfindung ist definiert als der Wert, welcher für entsprechenden Stoff bei 25 °C nach fünf Minuten Rühren mit einer gemäß ISO 4319 (1977) kalibrierten pH-Elektrode gemessen wird.

Die in den erfindungsgemäßen Zusammensetzungen enthaltenen Rhamnolipide liegen aufgrund des gegebenen pH-Wertes mindestens teilweise als Salz vor.

In erfindungsgemäß bevorzugten Zusammensetzungen sind die Kationen der enthaltenen Rhamnolipid-Salze, ausgewählt aus der Gruppe umfassend, bevorzugt bestehend aus, Li⁺, Na⁺, K⁺, Mg²⁺, Ca²⁺, Al³⁺, NH₄⁺, primäre Ammoniumionen, sekundäre Ammoniumionen, tertiäre Ammoniumionen und quaternäre Ammoniumionen.

Beispielhafte Vertreter von geeigneten Ammoniumionen sind Tetramethylammonium, Tetraethylammonium , Tetrapropylammonium, Tetrabutylammonium und [(2-Hydroxyethyl)trimethylammonium] (Cholin) sowie die Kationen von 2-Aminoethanol (Ethanolamin, MEA), Diethanolamin (DEA), 2,2',2"-Nitrilotriethanol (Triethanolamin, TEA), 1-Aminopropan-2-ol (Monoisopropanolamin), Ethylendiamin, Diethylentriamin, Triethylentetramin, Tetraethylenepentamine, 1,4-Diethylendiamin (Piperazin), Aminoethylpiperazin und Aminoethylethanolamin.

Es können auch Mischungen der vorgenannten Kationen als Kationen der enthaltenen Rhamnolipid-Salze erfindungsgemäß vorliegen.

Besonders bevorzugte Kationen, sind ausgewählt aus der Gruppe umfassend, bevorzugt bestehend aus Na⁺, K⁺, NH₄⁺ und das Triethanolammonium-Kation.

Die Gesamtmenge der vorgenannten Kationen macht bevorzugt 70 Gew.-% bis 99 Gew.-%, besonders bevorzugt 80 Gew.-% bis 90 Gew.-%, aller in der Zusammensetzung enthaltenen Kationen ohne H⁺ und H₃O⁺ aus.

Bevorzugte erfindungsgemäße Zusammensetzungen enthalten 50 Gew.-% bis 99 Gew.-%, bevorzugt 70 Gew.-% bis 95 Gew.-%, besonders bevorzugt 85 Gew.-% bis 90 Gew.-%, Rhamnolipid-Anionen, wobei sich die Gew.-% auf alle in der Zusammensetzung enthaltenen Anionen ohne OH⁻ beziehen.

Erfindungsgemäße Zusammensetzungen sind bevorzugt, die dadurch gekennzeichnet sind, dass sie das Rhamnolipid in einer Menge von 0,5 Gew.-% bis 70 Gew.-%, bevorzugt von 2 Gew.-% bis 60 Gew.-% , besonders bevorzugt von 3 Gew.-% bis 50 Gew.-%, enthalten, wobei sich die Gewichtsprozente auf die Gesamtzusammensetzung beziehen.

In einer bevorzugten Ausführungsform der erfindungsgemäßen Zusammensetzungen handelt es sich um Konzentrate, die das Rhamnolipid in einer Menge von 12 Gew.-% bis 70 Gew.-%, bevorzugt von 15 Gew.-% bis 60 Gew.-% , besonders bevorzugt von 20 Gew.-% bis 50 Gew.-%, enthalten, wobei sich die Gewichtsprozente auf die Gesamtzusammensetzung beziehen. Diese Konzentrate weisen bevorzugt eine Viskosität von 0,2 Pas bis 10 Pas, bevorzugt von 1,2 Pas bis 5Pas, besonders bevorzugt von 1,5 Pas bis 3 Pas, gemessen in einem Rheometer bei 25 °C und einer Scherrate von 10 s⁻¹, auf.

In einer alternativen bevorzugten Ausführungsform der erfindungsgemäßen Zusammensetzungen handelt es sich um gebrauchsfertige Formulierungen, die das Rhamnolipid in einer Menge von 0,5 Gew.-% bis 10 Gew.-%, bevorzugt von 2,5 Gew.-% bis 8 Gew.-% , besonders bevorzugt von 3 Gew.-% bis 7 Gew.-%, enthalten, wobei sich die Gewichtsprozente auf die Gesamtzusammensetzung beziehen.

Eine erfindungsgemäß bevorzugte Zusammensetzung ist dadurch gekennzeichnet, dass sie eine Mischung von Rhamnolipiden enthält, wobei in der Mischung das Gewichtsverhältnis von di-Rhamnolipiden zu mono-Rhamnolipiden größer als 51:49, bevorzugt größer 75:25, besonders bevorzugt 97:3, insbesondere größer 98:2 ist.

Erfindungsgemäß bevorzugte Zusammensetzung ist dadurch gekennzeichnet, dass die Rhamnolipid-Mischung
51 Gew.-% bis 95 Gew.-%, bevorzugt 70 Gew.-% bis 90 Gew.-%, besonders bevorzugt 75 Gew.-% bis 85 Gew.-%, diRL-C10C10 und
0,5 Gew.-% bis 9 Gew.-%, bevorzugt 0,5 Gew.-% bis 3 Gew.-%, besonders bevorzugt 0,5 Gew.-% bis 2 Gew.-%, monoRL-C10C10
enthält,
wobei sich die Gewichtsprozente auf die Summe aller enthaltenen Rhamnolipide beziehen,

Erfindungsgemäß bevorzugte Zusammensetzung ist dadurch gekennzeichnet, dass die Rhamnolipid-Mischung neben den oben genannten diRL-C10C10- und monoRL-C10C10 Gehalten 0,5 Gew.-% bis 15 Gew.-%, bevorzugt 3 Gew.-% bis 12 Gew.-%, besonders bevorzugt 5 Gew.-% bis 10 Gew.-%, diRL-C10C12:1
enthält,
wobei sich die Gewichtsprozente auf die Summe aller enthaltenen Rhamnolipide beziehen.

Erfindungsgemäß bevorzugte Zusammensetzung ist dadurch gekennzeichnet, dass die Rhamnolipid-Mischung neben den oben genannten diRL-C10C10- und monoRL-C10C10 Gehalten 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und/oder, bevorzugt und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthält,
wobei sich die Gewichtsprozente auf die Summe aller enthaltenen Rhamnolipide beziehen.

Es kann vorteilhaft sein und ist daher bevorzugt, wenn die in der erfindungsgemäßen Zusammensetzung enthaltene Rhamnolipid-Mischung neben den oben genannten diRL-C10C10- und monoRL-C10C10 Gehalten
0,1 Gew.-% bis 25 Gew.-%, bevorzugt 2 Gew.-% bis 10 Gew.-%, besonders bevorzugt 4 Gew.-% bis 8 Gew.-%, diRL-C8C10,
enthält,
wobei sich die Gewichtsprozente auf die Summe aller enthaltenen Rhamnolipide beziehen.

Erfindungsgemäß besonders bevorzugte Zusammensetzung ist dadurch gekennzeichnet, dass die Rhamnolipid-Mischung neben den oben genannten diRL-C10C10- und monoRL-C10C10 Gehalten 0,5 Gew.-% bis 15 Gew.-%, bevorzugt 3 Gew.-% bis 12 Gew.-%, besonders bevorzugt 5 Gew.-% bis 10 Gew.-%, diRL-C10C12:1,
0,5 bis 25 Gew.-%, bevorzugt 5 Gew.-% bis 15 Gew.-%, besonders bevorzugt 7 Gew.-% bis 12 Gew.-%, diRL-C10C12,
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthält,
wobei sich die Gewichtsprozente auf die Summe aller enthaltenen Rhamnolipide beziehen.

Es ist überdies hinaus bevorzugt, wenn die in der erfindungsgemäßen Zusammensetzung enthaltene Rhamnolipid-Mischung Rhamnolipide der Formel monoRL-CX bzw. diRL-CX in nur kleinen Mengen enthält. Insbesondere enthält die erfindungsgemäße Mischungszusammensetzung bevorzugt
0 Gew.-% bis 5 Gew.-%, bevorzugt 0,001 Gew.-% bis 3 Gew.-%, besonders bevorzugt 0,01 Gew.-% bis 1 Gew.-%, diRLC10, wobei sich die Gewichtsprozente auf die Summe aller enthaltenen Rhamnolipide beziehen, und unter dem Begriff "0 Gew.-%" keine nachweisbare Menge zu verstehen ist.

Die erfindungsgemäßen Zusammensetzungen sind insbesondere bevorzugt wässrige Zusammensetzungen.

Die erfindungsgemäßen Zusammensetzungen weisen bevorzugt einen pH-Wert von 5,5 bis 6,9 bevorzugt von 5,6 bis 6,2, besonders bevorzugt von 5,6 bis 6,0, auf.

Verfahren zur Herstellung der entsprechenden Rhamnolipid-Mischungen werden zum Beispiel in der EP2786743 und EP2787065 offenbart.

Bei den in den erfindungsgemäßen Zusammensetzungen enthaltenen Siloxanen handelt es sich um Verbindungen mit Si-O-Si-Bindungen. In ihnen sind Siliziumatome nicht direkt, sondern über Sauerstoffatome miteinander verbunden.

Die Eigenschaften und die Synthese von Siloxanen wird in "Walter Noll, Chemie und Technologie der Silicone, Verlag Chemie GmbH, 2. Auflage, 1968", "S.J.Clarson, J.A. Semlyen, Siloxane Polymers,PTR Prentice Hall, ISBN 0-13-816315-4" oder auch in Silicones, Vulkan-Verlag Essen ISBN: 3-8027-2161-6" und in "Moretto, H.-H., Schulze, M. and Wagner, G. 2000. Silicones. Ullmann's Encyclopedia of Industrial Chemistry". beschreiben

Häufig unterscheidet man organomodifizierte Siloxane von sogenannten Silikonölen.

Silikonöle sind Polymere mit Siloxaneinheiten, die als organische Reste lediglich Methyl- oder Phenylgruppen tragen. Silikonöle finden in einer Vielzahl von technischen Prozessen Anwendung.

Einfache linear polymere Silikonöle sind aus Einheiten (R₂SiO)x aufgebaut und lassen sich durch die folgende allgemeine Formel beschreiben: 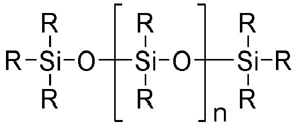 R = Me oder Ph,

In Anlehnung an die Systematik organischer Polymere kann man nach Noll die folgenden Gruppen unterscheiden:
(a) Lineare Polysiloxane (siehe oben)
(b) Verzweigte Polysiloxane: Diese enthalten als verzweigende Bausteine trifunktionelle oder tetrafunktionelle Siloxan-Einheiten. Die Verzweigungsstelle ist entweder in eine Kette oder einen Ring eingebaut.
(c) Cycl. Polysiloxane: Diese sind ringförmig aus difunktionellen Siloxan-Einheiten aufgebaut.
(d) Vernetzte Polymere: In dieser Gruppe sind kettenförmige oder ringförmige Mol. mit Hilfe von T-Einheiten und Q-Einheiten zu zweidimensionalen oder dreidimensionalen Netzwerken verknüpft.

Innerhalb jeder Polymeren-Gruppe lässt sich eine weitere Gliederung je nach der Art der am Silicium-Atom gebundenen Substituenten vornehmen. Das Siloxan-Gerüst kann mit verschiedenartigen Kohlenwasserstoff-Resten beladen sein, es kann außerdem Silicium-funktionelle oder organofunktionelle Gruppen oder beide zugleich enthalten. Dementsprechend ist eine Unterteilung der Polymeren-Gruppen in nichtfunktionelle und in Silicium-funktionelle oder organofunktionelle Polysiloxane zweckmäßig.

Die Siloxane können je nach Kettenlänge, Verzweigungsgrad und Substituenten niedrigviskos bis hochviskos oder fest sein.
Ist der Rest R in 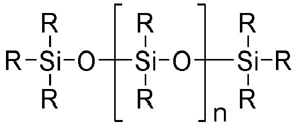 eine Alkylgruppe mit zwei oder mehr C-Atomen spricht man auch von Silikonwachsen. Diese lassen sich zum Beispiel durch die Hydrosilylierung von Olefinen mit SiH-funktionellen Siloxanen herstellen, wie es in der oben aufgeführten Literatur aufgezeigt ist. Silikonwachse werden zum Beispiel in Personal-care-Formulierungen eingesetzt. Typische Alkylreste sind Octyl, Dodecyl oder Hexadecyl.

Eine weitere Klasse von Siloxanen sind die Polydiethylsiloxane, bei denen Ethylgruppen an die Siliziumatome gebunden sind. Typischer Vertreter dieser Stoffklasse werden mit der CAS-Nummer 63148-61-8 beschrieben und finden ebenfalls Anwendung als Schmierstoff.

Des Weiteren gibt es eine Vielzahl von Siloxanen mit fluorierten Alkyresten, die zum Beispiel als Schmierstoffe eingesetzt werden.

Eine weitere Klasse von geeigneten Siloxanen sind Siloxane aufweisend funktionelle Gruppen, wie zum Beispiel Aminofunktionen. Derartige Siloxane werden unter anderen mit der CAS-Nummer 99363-37-8 oder der CAS-Nummer 7150-79-3 beschrieben. Diese beiden Stoffklassen finden Anwendung in Shampoos oder Spülungen für Haare.

Es können eine Vielzahl weitere funktionelle Gruppen an ein Siloxangerüst gebunden werden. Zudem können Mischungen verschiedener Funktionalitäten an ein Polymerrückgrat gebunden werden. In der oben aufgeführten Literatur sind verschiedene Wege hierfür beschrieben. Herauszuheben ist dabei die Hydrosilylierung, bei der SiH-funktionelle Siloxane mit ungesättigten organischen Verbindungen umgesetzt werden.

Alle oben gelisteten Siloxane lassen sich in den erfindungsgemäßen Siloxanen einsetzen.

Bevorzugt werden organomodifizierte Siloxane eingesetzt. Besonders bevorzugt handelt es sich bei diesen um Polyethersiloxane und insbesondere bevorzugt um Polyethersiloxane der allgemeinen Formel II.

Polyethersiloxane sind Siloxane, die sowohl Siloxaneinheiten, als auch Polyethereinheiten aufweisen. Siloxaneinheiten sind Einheiten der allgemeinen Formel -Si(R^{S1})₂O. Dabei ist R^{S1} ein Kohlenwasserstoffrest mit 1 - 20-Kohlenstoffatomen, bevorzugt ist R^{S1} eine Methylgruppe.

Insbesondere bevorzugt sind Polyethersiloxane:
Mₐ M¹_{b} D_{c} D¹_{d} Tₑ Q_{f}
Formel II 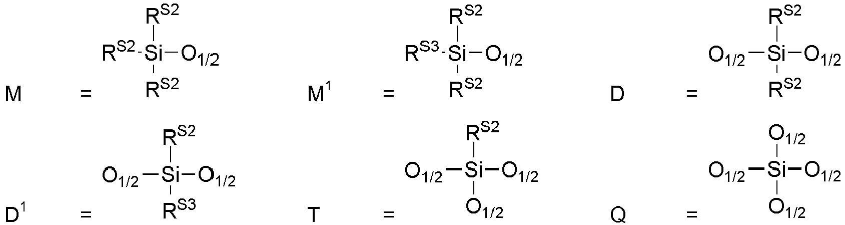
   a = 0 - 20, bevorzugt 0 - 10, insbesondere 2,
   b = 0 - 20, bevorzugt 0 - 10, insbesondere 0,
   c = 3 - 2000, bevorzugt 5 - 1000, insbesondere 10 - 500,
   d = 0 - 50, bevorzugt 1 - 30, insbesondere 1,5 - 15,
   e = 0 - 20, bevorzugt 0 - 10, insbesondere 0,
   f = 0 - 20, bevorzugt 0 - 10, insbesondere 0,
   dabei gilt als Einschränkung a + b ≥ 2 sowie N = a + b + c + d + e + f ≤ 2050, b + d ≥ 1
   R^{S2} = unabhängig voneinander gleiche oder verschiedene Alkylreste mit 1 - 16 Kohlenstoffatomen oder Arylreste mit 6 - 16 Kohlenstoffatomen oder H oder -OR^{S4}, bevorzugt Methyl, Ethyl, Phenyl, Octyl, Dodecyl oder H, insbesondere Methyl, Dabei ist R^{S4} unabhängig voneinander gleiche oder verschiedene Alkylreste mit 1 - 16 Kohlenstoffatomen oder Arylreste mit 6 - 16 Kohlenstoffatomen oder H,
   R^{S3} = unabhängig voneinander gleiche oder verschiedene Polyetherreste, bevorzugt gleiche oder verschiedene Polyetherreste der allgemeinen Formel III 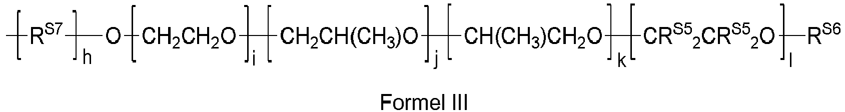 R^{S5} = gleiche oder verschiedene Alkylreste 1 bis 18 Kohlenstoffatomen, die gegebenenfalls Etherfunktionen besitzen, oder Arylreste mit 6 - 18 Kohlenstoffatomen , die gegebenenfalls Etherfunktionen besitzen, oder H, bevorzugt H, Ethyl und Benzyl,
   R^{S6} = gleiche oder verschiedene Reste aus der Gruppe: R^{S8}, H, -C(O)R^{S8}, bevorzugt Methyl, Butyl, H oder -C(O)Me,
   R^{S7} = unabhängig voneinander gleiche oder verschiedene divalente organische Reste, bevorzugt gleiche oder verschiedene divalente organische Reste mit 2 - 30 Kohlenstoffatomen, die gegebenenfalls durch Etherfunktionen unterbrochen sind und gegebenenfalls OH-Funktionen tragen, bevorzugt -(CH₂)₃ -
   R^{S8} = unabhängig voneinander gleiche oder verschiedene Alkylreste mit 1 - 16 Kohlenstoffatomen oder Arylreste mit 6 - 16 Kohlenstoffatomen,
   - h: = 0 oder 1,
   - i: = 0 - 200, bevorzugt 0 - 100, insbesondere bevorzugt 0 - 50,
   - j: = 0 - 200, bevorzugt 0 - 100, insbesondere bevorzugt 0 - 50,
   - k: = 0 - 200, bevorzugt 0 - 100, insbesondere bevorzugt 0 - 50,
   - l: = 0 - 80, bevorzugt 0 - 40, insbesondere bevorzugt 0,
   mit der Maßgabe i + j + k + I ≥ 3.

Zur Beschreibung der Siloxane wird hier eine Schreibweise analog der Literatur: Walter Noll, Chemie und Technologie der Silicone, Verlag Chemie GmbH, 2. Auflage, 1968, gewählt. Die erfindungsgemäßen Polyethersiloxane besitzen verschiedenen Siloxaneinheiten die im Molekül verschiedenartig miteinander kombiniert sein können.

Weiterhin wird die Beschreibung von Siloxanen mit M, D, T und Q-Einheiten im Buch Silicones, (Silicones, G. G. Freeman, The Plastic Institute, 1962, page 22-23.) beschrieben.

Die Zusammensetzung der Siloxaneinheiten ergibt sich unter der Berücksichtigung der Tatsache, dass jedes Sauerstoffatom als Brückenglied zwischen je zwei Siliziumatomen fungiert, und demgemäß jedem Siliziumatom nur zur Hälfte zuzurechnen ist. Die verschiedenen Siloxaneinheiten sind über über 2 halbe Sauerstoffatome (-O_{1/2}O_{1/2}-) Gruppen miteinander verknüpft, wodurch eine Sauerstoffbrücke (-O-) dargestellt wird.

Von besonderer Bedeutung sind Polyethersiloxane mit Polyetherresten, die über SiC-Funktionen an ein Siloxanrückgrad geknüpft sind. Sie lassen sich durch die Hydrosilylierung von Polyethern mit endständigen CC-Doppebindungen mit SiH-Siloxanen herstellen. CC-Doppelbindungshaltige Polyether lassen sich zum Beispiel durch die Alkoxylierung von Allylalkohol herstellen und werden als Allyloxypolyethyleneglykole vertrieben. Typische Vertreter dieser Stoffklasse werden beispielsweise mit Hilfe der CAS-Nummer: 27274-31-3, 9042-19-7 und 9041-33-2 beschrieben. Die Herstellung von Polyethersiloxanen mittels Hydrosilylierung ist ein bekannter Prozess und wird vielfach in der Literatur beschrieben, zum Beispiel in den Buch "Chemie und Technologie der Silicone", Verlag Chemie, 1960, Seite 43. Als Katalysatoren für die Hydrosilylierung werden üblicherweise Platin-Verbindungen eingesetzt. In der betrieblichen Praxis haben sich hierfür Hexachloroplatinsäure und der Karstedt-Katalysator bzw. Formulierungen dieser Verbindungen durchgesetzt.

Die in den erfindungsgemäßen Zusammensetzungen enthaltenen Siloxane sind bevorzugt in einer Menge von 0,1 ppm bis 5000 ppm, bevorzugt von 1 ppm bis 4000 ppm, besonders bevorzugt von 10 ppm bis 2000 ppm, enthalten, wobei sich die ppm auf die Gesamtzusammensetzung beziehen. In der bereits oben genannten, bevorzugten Ausführungsform der erfindungsgemäßen Zusammensetzungen "Konzentrate", ist das Siloxan bevorzugt in einer Menge von 0,5 ppm bis 5000 ppm, von 5 ppm bis 4000 ppm, besonders bevorzugt von 10 ppm bis 2000 ppm, enthalten, wobei sich die ppm auf die Gesamtzusammensetzung beziehen.

In der bereits oben genannten alternativen bevorzugten Ausführungsform der erfindungsgemäßen Zusammensetzungen "gebrauchsfertige Formulierungen", ist das Siloxan bevorzugt in einer Menge von 0,1 bis 1000 ppm, bevorzugt von 1 bis 500 ppm, besonders bevorzugt von 2 bis 200 ppm, enthalten, wobei sich die ppm auf die Gesamtzusammensetzung beziehen.

Die erfindungsgemäßen Zusammensetzungen lassen sich vorteilhaft in Wasch- und Reinigungsmitteln und insbesondere in kosmetischen Formulierungen einarbeiten.

Somit ist ein weiterer Gegenstand der vorliegenden Erfindung die Verwendung der erfindungsgemäßen Zusammensetzungen zur Herstellung von Formulierungen, insbesondere von kosmetischen Formulierungen,
sowie die Formulierungen, insbesondere kosmetische Formulierungen, welche die erfindungsgemäße Zusammensetzungen enthalten.

Bevorzugte erfindungsgemäße Formulierungen enthalten neben den erfindungsgemäßen Zusammensetzungen mindestens ein weiteres Tensid, wobei beispielsweise anionische, nichtionische, kationische und/oder amphotere Tenside eingesetzt werden können. Bevorzugt sind aus anwendungstechnischer Sicht Mischungen aus anionischen und nichtionischen Tensiden. Der Gesamttensidgehalt der wässrigen Formulierung beträgt vorzugsweise 5 bis 60 Gew.-% und besonders bevorzugt 15 bis 40 Gew.-%, bezogen auf die gesamte Formulierung.

Die erfindungsgemäßen Formulierungen können des Weiteren mindestens eine zusätzliche Komponente enthalten, ausgewählt aus der Gruppe der
Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Geruchsabsorber,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Die erfindungsgemäßen Zusammensetzungen sowie die erfindungsgemäßen Formulierungen enthaltend die erfindungsgemäßen Zusammensetzungen lassen sich vorteilhaft zur Reinigung von Oberflächen verwenden, z.B. zur Reinigung von Leder. Bei dieser Form der erfindungsgemäßen Verwendung ist die Oberfläche bevorzugt die Oberfläche eines Lebewesens, insbesondere eines Menschen, wobei solche Oberflächen besonders bevorzugt ausgewählt sind aus Haut und Haar.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Herstellung von Rhamnolipiden

Eine Fermentation mit einem die Rhamnolipid-Biosynthesegene RhIA, RhIB und RhIC enthaltenden *Pseudomonas putida* Stamm *pBBR1MCS2-Plac-rh*/*ABC-T-Ptac-rh*/*C-T,* deren Herstellung in US2014296168 beschrieben ist, wurde durchgeführt. Die Vorkultur im Schüttelkolben wurde wie in WO2012013554A1 beschrieben durchgeführt. Für die Hauptkultur kam ebenfalls ein Mineralmedium (M9) zum Einsatz. Die Fermentation wurde kohlenstofflimitiert über eine Glukosezufütterung in einem 2 Liter Fermenter geführt. Die Glukosezufütterung erfolgt anhand des Gelöstsauerstoffsignals. Der Gelöstsauerstoff wurde bei 20 % Sättigung über die Rührerdrehzahl reguliert. Der pH-Wert wird über eine pH Elektrode und Zugabevon 2M Schwefelsäure bzw. einer 20 Gew.-% Ammoniaklösung auf 7 reguliert. Um das Überschäumen der Fermentationsbrühe zu verhindern, wurde der Entschäumer DOW Corning 1500 bei Bedarf zudosiert. Die Fermentation wurde über 4 Tage bis zu einer Biotrockenmasse von 15 g/l geführt. Die Rhamnolipidkonzentration wurde über HPLC ermittelt und betrug 9,8 g/l. Nach Abtrennen der Zellen mittels Zentrifugation bei 10.000 g wurde die Fermentationsbrühe durch Zugabe konzentrierter H₂SO₄ auf einen pH-Wert von 3,1 eingestellt. Durch erneute Zentrifugation bei 500 g wurde ein pastöses Feststoff-Konzentrat mit einem RL-Anteil von 45 Gew.-% und mit einer Viskosität von > 10.000 mPas erhalten. Unter ständigem Rühren wurde eine 50 gew.-%ige wässrige KOH Lösung zur pastösen Suspension des aufkonzentrierten Rhamnolipidpräzipitats gegeben und ein pH-Wert von 6 eingestellt. Hierbei kam es zur Verflüssigung der pastösen Masse die mit einem starken Viskositätsabfall einherging. Aus der Suspension wurde eine klare Lösung. Durch Zugabe von Wasser wurde die Lösung auf einen Aktivgehalt von 35 Gew.-% eingestellt. Die Rhamnolipid-Reinheit betrug > 90 Gew.-% bezogen auf die Trockenmasse.

Mittels HPLC nachgewiesene Rhamnolipidspezies waren:

| | |
|---|---|
| RL gesamt [%] (HPLC) | **91** |
| diRL-C8C10 | 13.9 |
| monoRL-C8C10 | 0.51 |
| diRL-C10C10 | 61.4 |
| monoRL -C10C10 | 1.4 |
| diRL-C10C12:1 | 5.9 |
| diRL-C10C12 | 5.5 |
| other RL | 2.2 |

### Beispiel 2: Herstellung von mono-Rhamnolipiden

Die wie oben beschrieben hergestellte, 35 Gew.-% Rhamnolipidlösung wurde durch Zugabe von Wasser auf 1 % verdünnt. Zwei Liter dieser Lösung wurden auf 50 °C erwärmt. Unter leichtem Rühren wurden 200 Units einer thermostabilen Rhamnosidase (ThermoActiveTM Rhamnosidase A, Prokazyme) zugegeben und die Reaktion über Nacht durchgeführt. Nach 20 h wurde eine Probe der Lösung mittels HPLC analysiert. Das di-Rhamnolipid war vollständig zu mono-Rhamnolipid und Rhamnose umgesetzt worden. Anschließend wurde das Enzym bei 80 °C eine Stunde inaktiviert. Dann wurde der gesamte Ansatz gefriergetrocknet. Das gefriergetrocknete Produkt wurde durch Wasserzugabe auf einen mono-Rhamnolipid Aktivgehalt von 35 Gew.-% eingestellt.

### Beispiel 3: eingesetzte Siloxane

Siloxan 1, organomodifiziert
MD₈₀D¹₈M
R^{S2} = Me, 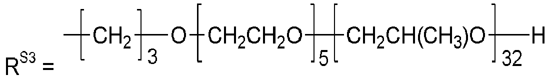 Siloxan 2 (Silikonöl)
MD₇₈M

Siloxan 3, organomodifiziert
M¹D₁₈M¹
R^{S2} = Me, 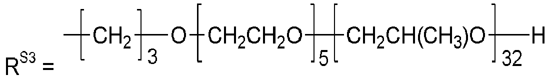 Siloxan 4, organomodifiziert
MD₈₀D¹₈M
R^{S2} = Me, 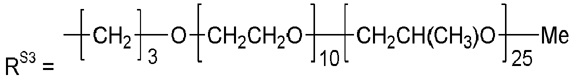 Siloxan 5, organomodifiziert
M¹D₁₈M¹
R^{S2} = Me,

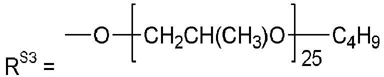

Siloxan 1, 3 und 4 sind Polyethersiloxane bei denen der Polyetherrest über eine SiC-Gruppe an das Siloxanrückgrat gebunden ist. Diese wurden analog dem Beispiel 1 in EP1520870 hergestellt

Siloxan 5 ist ein Polyethersiloxan bei dem der Polyetherrest über eine SiOC-Funktion an das Siloxanrückgrat gebunden ist. Diese Verbindung wurde wie in Vergleichsbeispiel A 1.b aus der EP1627892 beschrieben hergestellt.

Siloxan 2 ist ein Polydimethylsiloxan, welches von Gelest bezogen wurde (Product Code: DMS-T21)

### Beispiel 4: Herstellung der Rhamnolipid-Siloxan Zusammensetzungen

Zu der 35 Gew.-% Rhamnolipidlösung (Beispiel 1) wurden die Siloxane unter langsamen Rühren auf dem Magnetrührer bei 50 °C zugetropft und eine Stunde weiter gerührt. Durch anschließende Zugabe von Wasser wurde der Rhamnolipidgehalt auf 30 Gew.-% eingestellt.

**Tabelle 1: Wässrige Rhamnolipid-Zusammensetzung (Angaben in Gew.-% in Wasser) mit zunehmendem Anteil an Siloxan**

| Zusammensetzung | 1a | 1b* | 1c* | 1d* | 1e* | 1f |
|---|---|---|---|---|---|---|
| RL/Siloxan Verhältnis | - | 3000:1 | 1000:1 | 500:1 | 250:1 | 50:1 |
| RL Gehalt | 30,0 % | 30,0 % | 30,0 % | 30,0 % | 30,0 % | 30,0 % |
| Siloxan 1 | | 0,01 % | 0,03 % | 0,06 % | 0,12 % | 0,6 % |

| | | | | | | |
|---|---|---|---|---|---|---|
| *=erfindungsgemäß | | | | | | |

**Tabelle 2: Wässrige Rhamnolipid- Zusammensetzung mit verschiedenen Siloxanen (Angaben in Gew. % in Wasser)**

| Zusammensetzung | 2a | 2b | 2c* | 2d | 2e* | 2f | 2g* |
|---|---|---|---|---|---|---|---|
| RL/Siloxan Verhältnis | - | 50:1 | 1000:1 | 50:1 | 1000:1 | 50:1 | 1000:1 |
| RL Gehalt | 30,0 % | 30,0 % | 30,0 % | 30,0 % | 30,0 % | 30,0 % | 30,0 % |
| Siloxan 1 Gehalt | | 0,6 % | 0,03 % | | | | |
| Siloxan 2 Gehalt | | | | 0,6 % | 0,03 % | | |
| Siloxan 3 Gehalt | | | | | | 0,6 % | 0,03 % |

### Fortsetzung Tabelle 2

| Zusammensetzung | 2h | 2i* | 2j | 2k* |
|---|---|---|---|---|
| RL/Siloxan Verhältnis | 50:1 | 1000:1 | 50:1 | 1000:1 |
| RL Gehalt | 30,0 % | 30,0 % | 30,0 % | 30,0 % |
| Siloxan 4 Gehalt | 0,6 % | 0,03 % | | |
| Siloxan 5 Gehalt | | | 0,6 % | 0,03 % |

Um verschiedenen Verhältnisse an di- zu mono-Rhamnolipid herzustellen, wurden die Rhamnolipidlösungen aus Beispiel 1 und Beispiel 2 in verschiedenen Verhältnissen gemischt. Dann wurde wie oben beschrieben Siloxan zugegeben und durch Wasserzugabe der Gesamtrhamnolipidgehalt auf 30 Gew.-% eingestellt.

**Tabelle 3: Wässrige Rhamnolipid- Zusammensetzung mit Siloxan und verschiedenen mono-/di-Rhamnolipid Verhältnissen (Angaben in Gew.-% in Wasser)**

| Zusammensetzung | 3a | 3b* | 3c | 3d* | 3e | 3f* | 3g | 3h* |
|---|---|---|---|---|---|---|---|---|
| RL/Siloxan Verhältnis | 50:1 | 1000:1 | 50:1 | 1000:1 | 50:1 | 1000:1 | 50:1 | 1000:1 |
| di-Rhamnolipid pH =6 | 22,5 % | 22,5 % | 15,0 % | 15,0 % | 7,5 % | 22,5 % | 0% | 0% |
| mono-Rhamnolipid pH 6 | 7,5 % | 7,5 % | 15,0 % | 15,0 % | 7,5 % | 67,5 % | 30,0 % | 30,0 % |
| Siloxan 1 | 0,6 % | 0,03 % | 0,6 % | 0,03 % | 0,6 % | 0,03 % | 0,6 % | 0,03 % |

### Beispiel 5: Anwendungstest Leder

Velourlederstreifen (15 x 5 cm) wurden mit feinem Prüfstaub (KSL Staubtechnik GmbH) gemäß DIN EN 60068-2-68 bedeckt. Anschließend wurde der Staub soweit wie möglich durch Ausschütteln von den Lederstreifen entfernt. Die in Tabelle 2 beschriebenen Zusammensetzungen wurden durch Wasserzugabe auf einen Rhamnolipidgehalt von 10 Gew.-% verdünnt. Die Lösungen wurden auf einem Magnetrührer gerührt und jeweils ein verschmutzen Verlourlederstreifen für 20 Sekunden in eine der Lösungen gelegt. Anschließend wurden die Verlourlederstreifen 20 Sekunden unter fließendem Leitungswasser gespült und dann in einem Trockenschrank bei 40 °C für 24 h getrocknet. Ein Gruppe von 10 Testpersonen beurteilte den Erhalt der Velourlederstruktur im Vergleich zu einem nicht behandelten Velourledersteifen und den Reinigungseffekt, d.h. die Staubentfernung im Vergleich zu einem verschmutzten, nicht gereinigten Velourlederstück.

**Tabelle 4: Ergebnisse der Untersuchungen zur Reinigung von Velour**

| Zusammensetzung | 1a | 1b* | 1c* | 1d* | 1e* | 1f |
|---|---|---|---|---|---|---|
| Erhalt der Wildlederstruktur (1 = unverändert, 5 = stark verändert) | 2,4 | 1,5 | 1,3 | 1,6 | 2,0 | 3,2 |
| Staubentfernung (1 = vollständig, 5 = keine Reinigungswirkung) | 3,3 | 2,6 | 2,1 | 2,0 | 2,2 | 2,5 |

### Beispiel 6: Anwendungstest Geruchsreduktion und Hautgefühl

Zur Bewertung der Geruchsreduzierung auf der Haut nach der Reinigung mit wässrigen, tensidischen Formulierungen enthaltend Rhamnolipid mit verschiedenen Anteilen von Siloxankomponenten wurden von einem geschulten Geruchspanel (umfassend wenigstens 10 Prüfpersonen) Geruchstests durchgeführt, die im Folgenden beschrieben werden. 50 g gewürfelte Küchenzwiebel wurden mit Hilfe eines Pürierstabes auf Stufe 2 für 2 Minuten homogenisiert und die erhaltene Paste mit einer 0,5%igen Lösung von Tego Betain F 50 (Evonik Industries AG) in voll entsalztem Wasser auf 1000 ml aufgefüllt. Die Suspension wurde anschließend mit Hilfe eines Magnetrührers 30 Minuten bei 680rpm und bei 25°C mit einem Rührkern (60mm Länge, 10mm Durchmesser) in einem mit einem Uhrglas abgedeckten 2L-Becherglas (flache Form) gerührt und nachfolgend über ein 190 µm Schnellsieb der Firma Erich Drehkopf GmbH filtriert. Je 2ml der erhaltenen wässrigen Zwiebellösung wurden mit einer Pipette auf die Handinnenflächen appliziert und mit 10 gleichmäßigen Reibbewegungen auf dem Handteller verteilt. Die Hände wurden anschließend für 60 Sekunden bei Raumtemperatur und einer Luftfeuchtigkeit von (50 +/- 10)% trocknen gelassen. Dazu wusch sich die Gruppe aus wenigstens 10 trainierten Prüfpersonen nach einer genau definierten Vorgehensweise mit den in Tabelle 2 und Tabelle 3 beschriebenen Zusammensetzungen die Hände und bewertete den Geruch der Handinnenflächen direkt nach dem Händewaschen und nach 5 Minuten anhand einer Notenskala von 0 (kein Geruch wahrnehmbar) bis 3 (sehr starker Geruch, sehr unangenehm). Zusätzlich wurde das Hautgefühl anhand einer Notenskala von 1 (sehr gut) bis 5 (sehr schlecht) beurteilt. Zur Bewertung der Geruchsreduzierung von erfindungsgemäßen Produktbeispielen wurden Geruchspaneltests auch im Vergleich zum Sekundärtensid Cocamidopropyl Betaine durchgeführt, das in der Industrie als universell einsetzbares Tensid weit verbreitet ist.

**Tabelle 5: Ergebnis Handwaschtest**

| Zusammensetzung | Cocoamidopropyl Betain (30 %) | 2a | 2b | 2c* | 2e* | 2g* | 2i* |
|---|---|---|---|---|---|---|---|
| Geruch (0 = kein Geruch wahrnehmbar bis 3 = sehr starker Geruch, sehr unangenehm | 2,7 | 2,3 | 2,4 | 1,2 | 1,2 | 0,7 | 1,5 |
| Hautgefühl während des Waschens (1 = sehr gut, 5 = sehr schlecht) | 2,7 | 2,3 | 1,6 | 1,7 | 2,0 | 1,8 | 2,1 |

**Fortsetzung Tabelle 5**

| Zusammensetzung | 2k* | 3b* | 3d* | 3f* | 3h* |
|---|---|---|---|---|---|
| Geruch (0 = kein Geruch wahrnehmbar bis 3 = sehr starker Geruch, sehr unangenehm | 1,4 | 0,9 | 1,1 | 1,3 | 1,2 |
| Hautgefühl während des Waschens (1 = sehr gut, 5 = sehr schlecht) | 2,0 | 2,3 | 1,9 | 2,1 | 1,7 |

Anhand der Messergebnisse wird ersichtlich, dass das Händewaschen mit den erfindungsgemäßen Formulierungen unter Verwendung der erfindungsgemäßen Zusammensetzungen die höchste Geruchsreduzierung bewirkt, wobei ein gutes Hautgefühl vorliegt.

### Formulierungsbeispiele

### "Zusammensetzungen" sind die der Tabellen 1 bis 3 oben.

Laurylethersulfat-basierte Systeme:

### Feuchtigkeitspendendes Hautreinigungsmittel

| | | |
|---|---|---|
| A | Sodium Laureth Sulfate | 8,0% |
| | Zusammensetzung 1e* | 2,0% |
| | Parfum | q.s. |
| B | Water | ad 100% |
| | Hydroxypropyl Methylcellulose | 1,2% |
| | Cocamidopropyl Betaine | 3,0% |
| | PPG-3 Myristyl Ether | 1,0% |
| | Glycol Distearate | 2,0% |
| | PEG-200 Hydrogenated Glyceryl Palmate (and) PEG-7 Glyceryl Cocoate | 1,0% |
| | Preservative | q.s. |
| | Citric Acid, 30% | ad pH 5,5 |

### Körperreinigungsmittel mit Perleffekt

| | |
|---|---|
| Sodium Laureth Sulfate | 9,0% |
| Zusammensetzung 1d* | 2,5% |
| Water | ad 100% |
| Cocamidopropyl Betaine | 1,5% |
| Glycol Distearate (and) Laureth-4 (and) Cocamidopropyl Betaine | 2,0% |
| PEG-18 Glyceryl Oleate/Cocoate | 1,5% |
| NaCl | 0,5% |
| Parfum | q.s. |
| Preservative | q.s. |

### Handreinigungspaste

| | |
|---|---|
| Water | ad 100% |
| Laureth-5 | 5,0% |
| Juglans Regia | 3,0% |
| Sodium Laureth Sulfate | 5,0% |
| Zusammensetzung 2c* | 2,0% |
| Bentonite | 1,0% |
| Sodium Cocoamphoacetate | 0,7% |
| Oleic Acid | 0,5% |
| C12-13 Alkyl Lactate | 0,5% |
| Aloe Barbadensis | 0,3% |
| Sodium Chloride | 0,3% |
| PEG-14M | 0,2% |
| Citric Acid | ad pH 6,0 |
| Preservative, Parfum | q.s. |

### Hautreinigungsmittel

| | |
|---|---|
| Water | ad 100% |
| Ammonium Laureth Sulfate | 5,5% |
| Cocamidopropyl Betaine | 2,5% |
| Zusammensetzung 2e* | 1,5% |
| Sorbitol | 1,2% |
| Cocamide Methyl MEA | 0,7% |
| PEG-7 Glyceryl Cocoate | 0,6% |
| Sodium Cocoyl Alaninate | 0,8% |
| Sodium Chloride | 0,7% |
| DMDM Hydantoin | 0,1% |
| Disodium EDTA | 0,1% |
| Santalum Album Extract (Extract) | 0,1% |
| Lactic Acid, 90% | ad pH 5,5 |
| Preservative, Parfum | q.s. |

### Alkylsulfat-basierte Systeme:

### Pflegendes Körperreinigungsmittel

| | |
|---|---|
| Water | ad 100% |
| Ammonium Lauryl Sulfate | 5,0% |
| Lauryl Glucoside | 3,0% |
| Cocamidopropyl Betaine | 2,5% |
| Zusammensetzung 3b* | 2,0% |
| Lauroyl Sarcosine | 0,7% |
| Bambusa Arundinacea Extract | 1,0% |
| Citrus Grandis Extract | 1,0% |
| Alcohol | 0,7% |
| Hydroxypropyl Guar Hydroxypropyltrimonium Chloride | 0,3% |
| Citrus Medica Limonum Oil | 0,3% |
| Preservative, Parfum | q.s. |

### Milde Waschlotion

| | |
|---|---|
| Water | ad 100% |
| Sodium Coco Sulfate | 5,5% |
| Glycerin | 3,5% |
| Zusammensetzung 3d* | 3,0% |
| Decyl Glucoside | 1,5% |
| Alcohol | 1,0% |
| Xanthan Gum | 1,0% |
| Bellis Perennis Extract | 0,7% |
| Arnica Montana Extract | 0,7% |
| Chamomilla Recutita Extract | 0,5% |
| Disodium Cocoyl Glutamate | 0,5% |
| Sodium Cocoyl Glutamate | 0,3% |
| Preservative, Parfum | q.s. |

### Betain-basierte Systeme:

Mildes, sulfat-freies Körperreinigungsmittel

| | |
|---|---|
| Cocamidopropyl Betaine | 5,0% |
| Sodium Cocoamphoacetate | 4,0% |
| Zusammensetzung 3f* | 1,5% |
| Sucrose Cocoate | 1,5% |
| PEG-120 Methyl Glucose Dioleate | 2,0% |
| Polyquaternium-10 | 0,2% |
| Water | ad 100% |
| Citric Acid, 30% | ad pH 6,0 |
| Preservative, Parfum, Dyes | q.s. |

### Mildes, PEG-freies Körperreinigungsmittel

| | |
|---|---|
| Water | ad 100% |
| Cocamidopropyl Betaine | 5,5% |
| Lauryl Glucoside | 3,0% |
| Sodium Cocoamphoacetate | 3,0% |
| Zusammensetzung 1c* | 3,0 % |
| Hydroxypropyl Methylcellulose | 0,5% |
| Isostearamide MIPA (and) Glyceryl Laurate | 1,4% |
| Polyquaternium-7 | 0,5% |
| Citric Acid, 30% | ad pH 5,0 |
| Parfum, Preservative | q.s. |

### Amphoacetat-basierte Systeme:

### Körperreinigungsmittel, PEG- & Sulfat-frei

| | |
|---|---|
| Water | ad 100% |
| Sodium Cocoamphoacetate | 5,0% |
| Disodium Lauryl Sulfosuccinate | 1,2% |
| Zusammensetzung 1c* | 2,5% |
| Cocamidopropyl Betaine | 3,0% |
| Cocamidopropyl Betaine (and) Glyceryl Laurate | 1,0% |
| Citric Acid, 30% | ad pH 5,0 |
| Preservative, Parfum | q.s. |

### Shampoo, PEG- & Sulfat-frei

| | |
|---|---|
| Water | ad 100% |
| Sodium Cocoamphoacetate | 5,5% |
| Disodium Cocoyl Glutamate | 2,0% |
| Zusammensetzung 2g* | 2,0% |
| Polyquaternium-10 | 0,2% |
| Palmitamidopropyltrimonium Chloride | 1,0% |
| Isostearamide MIPA | 1,0% |
| Citric Acid, 30% | ad pH 5,5 |
| Preservative, Parfum | q.s. |

### APG-basierte Systeme:

### Mildes Körperreinigungsmittel

| | |
|---|---|
| Water | ad 100% |
| Lauryl Glucoside | 5,0% |
| Coco Glucoside | 2,0% |
| Zusammensetzung 2i* | 3,0% |
| Sucrose Cocoate | 1,5% |
| Cocamidopropyl Betaine | 4,0% |
| Carbomer | 1,0% |
| Citric Acid, 30% | ad pH 5,5 |
| Preservative, Parfum | q.s. |

### Duschgel

| | |
|---|---|
| Water | ad 100% |
| Zusammensetzung 2k* | 4,0% |
| Coco Glucoside | 4,0% |
| Glycerin | 3,0% |
| Disodium Cocoyl Glutamate | 2,0% |
| Sodium Cocoyl Glutamate | 2,0% |
| Polyglyceryl-10 Laurate | 1,0% |
| Glyceryl Caprylate | 1,0% |
| Rubus Idaeus (Raspberry) Fruit Extract | 0,3% |
| Sodium PCA | 0,2% |
| Xanthan Gum | 0,6% |
| Glyceryl Oleate | 0,3% |
| Phytic Acid | 0,1% |
| Citric Acid | ad pH 5,5 |
| Parfum | q.s. |

### Mildes Haar- & Körperreinigungsmittel, ECOCERT Inhaltsstoffe

| | |
|---|---|
| Lauryl Glucoside | 3,0% |
| Zusammensetzung 1c* | 2,0% |
| Cocamidopropyl Betaine (and) Glyceryl Laurate | 5,0% |
| Sorbitan Sesquicaprylate | 0,9% |
| Water | ad 100% |
| Cocamidopropyl Betaine | 4,0% |
| Citric Acid, 30 % | ad pH 5,5 |
| Preservative, Parfum | q.s. |

### Milder Reinigungsschaum

| | |
|---|---|
| Water | ad 100% |
| Glycine Soja Oil | 8,0% |
| Glycerin | 5,0% |
| Alcohol | 5,0% |
| Zusammensetzung 3h* | 4,0% |
| Coco Glucoside | 3,5% |
| Caprylic/Capric Triglyceride | 2,0% |
| Sodium Coco Sulfate | 2,0% |
| Sodium Lactate | 1,0% |
| Sodium Cocoyl Glutamate | 1,0% |
| Disodium Cocoyl Glutamate | 0,6% |
| Argania Spinosa Kernel Oil | 0,6% |
| Preservative, Parfum | q.s. |

### Mildes Körperreinigungsmittel, PEG- und Sulfat-frei

| | |
|---|---|
| Lauryl Glucoside | 3,5% |
| Zusammensetzung 3h* | 2,0% |
| Isostearamide MIPA (and) Glyceryl Laurate | 1,0% |
| Water | ad 100% |
| Coco Glucoside | 1,0% |
| Sodium Cocoamphoacetate | 3,0% |
| Cocoamidopropyl Betaine | 3,5% |
| Citric Acid, 30% | ad pH 5,5 |
| Preservative, Parfum | q.s. |

### Sulfonat-basiertes System:

### Sulfatfreies Körperreinigungsmittel

| | |
|---|---|
| Water | ad 100% |
| Sodium C14-16 Olefin Sulfonate | 4,0% |
| Zusammensetzung 1c* | 3,0% |
| Cocoamidopropyl Betaine | 3,0% |
| Methylhydroxyethylcellulose | 1,5% |
| Preservative, Parfum | q.s. |

### Sulfosuccinat-basierte Systeme:

### Mildes Hautreinigungsmittel

| | |
|---|---|
| Water | ad 100% |
| Disodium Laureth Sulfosuccinate | 3,5% |
| Zusammensetzung 1c* | 3,5% |
| Isostearamide MIPA (and) Glyceryl Laurate | 1,7% |
| PEG-7 Glyceryl Cocoate | 0,5% |
| Sodium Cocoamphoacetate | 3,0% |
| Palmitamidopropyltrimonium Chloride | 2,3% |
| Citric Acid, 30% | ad pH 6,0 |
| Preservative, Parfum, Dyes | q.s. |

### Milder Hautreinigungsschaum

| | |
|---|---|
| Water | ad 100% |
| Disodium PEG-5 Laurylcitrate Sulfosuccinate (and) Capryl/ Capramidopropyl Betaine | 5,5% |
| Zusammensetzung 1e* | 3,0% |
| Capryl/Capramidopropyl Betaine | 2,0% |
| Polyglyceryl-3 Caprate | 0,5% |
| Creatine | 0,2% |
| Hydroxypropyl Methylcellulose | 0,5% |
| Sodium Lactate (and) Sodium PCA (and) Glycine (and) Fructose (and) Urea (and) Niacinamide (and) Inositol (and) Sodium Benzoate (and) Lactic Acid | 1,0% |
| Preservative, Parfum | q.s. |

### Sarcosinat-basierte Systeme:

### Hautreinigungsmittel

| | |
|---|---|
| Water | ad 100% |
| Sodium Lauroyl Sarcosinate | 5,0% |
| Coco Betaine | 3,0% |
| Zusammensetzung 1e* | 3,0 % |
| Cocamide DEA | 4,5% |
| Cetrimonium Chloride | 0,3% |
| Steralkonium Chloride | 0,1% |
| Disodium EDTA | 0,2% |
| Citric Acid | ad pH 6,7 |
| Preservative, Parfum | q.s. |

### Hautreinigungsmittel

| | |
|---|---|
| Water | ad 100% |
| Sodium Lauroyl Sarcosinate | 5,0% |
| Zusammensetzung 1c* | 2,5% |
| Cocamidopropyl Betaine | 4,0% |
| Palmitamidopropyltrimonium Chloride | 0,5% |
| Polyquaternium-10 | 0,1% |
| Citric Acid | ad pH 5,1 |
| Preservative, Parfum | q.s. |

### anders-basierte Systeme (Sultain, Anisat, Isethionat, Glutamat, Glycinat):

### Duschgel

| | |
|---|---|
| Water | ad 100% |
| Sodium Lauroyl Methyl Isethionate | 4,5% |
| Zusammensetzung 1c* | 3,0% |
| Cocamidopropyl Betaine | 2,5% |
| Sodium Chloride | 2,5% |
| Glycerin | 1,5% |
| Polyglyceryl-4 Caprate | 0,6% |
| Sucrose Cocoate | 0,5% |
| Trisodium Ethylenediamine Disuccinate | 0,2% |
| Zinc Laurate | 0,1% |
| Salicylic Acid | 0,1% |
| Propylene Glycol | 0,1% |
| Aloe Barbadensis Leaf Juice | 0,1% |
| Sodium Hydroxide | 0,1% |
| Tocopherol | 0,1% |
| Citric Acid | ad pH 5,5 |
| Preservative, Parfum | q.s. |

### Schäumendes Körperreinigungsmittel

| | |
|---|---|
| Water | ad 100% |
| Sodium Cocoyl Glycinate | 6,0% |
| Zusammensetzung 1e* | 4,0% |
| Coco-betaine | 2,0% |
| Glycerin | 1,0% |
| Sodium Chloride | 1,0% |
| Acrylates/C10-30 Alkyl Acrylate Crosspolymer | 0,5% |
| Sodium Hydroxide | 0,4% |
| PEG-14M | 0,3% |
| Salicylic Acid | 0,1% |
| Polyquaternium-10 | 0,1% |
| Glycol Distearate | 0,2% |
| Citric Acid | ad pH 5,5 |
| Preservative, Parfum, Dyes | q.s. |

## Patentansprüche

1. Zusammensetzung enthaltend mindestens ein Rhamnolipid und mindestens ein Siloxan, **dadurch gekennzeichnet, dass** das Gewichtsverhältnis von Rhamnolipid zu Siloxan von 5.000.000 zu 1 bis 100 zu 1, bevorzugt von 500.000 zu 1 bis 1.000 zu 1, besonders bevorzugt von 25.000 zu 1 bis 2.500 zu 1 beträgt.

2. Zusammensetzung gemäß Anspruch 1, **dadurch gekennzeichnet, dass** sie das Rhamnolipid in einer Menge von 0,5 Gew.-% bis 70 Gew.-%, bevorzugt von 2 Gew.-% bis 60 Gew.-% , besonders bevorzugt von 3 Gew.-% bis 50 Gew.-%, enthält, wobei sich die Gewichtsprozente auf die Gesamtzusammensetzung beziehen.

3. Zusammensetzung gemäß Anspruch 1 oder 2, **dadurch gekennzeichnet, dass** sie das Rhamnolipid in einer Menge von 12 Gew.-% bis 70 Gew.-%, bevorzugt von 15 Gew.-% bis 60 Gew.-% , besonders bevorzugt von 20 Gew.-% bis 50 Gew.-%, enthalten.

4. Zusammensetzung gemäß mindestens einem der vorherigen Ansprüche, **dadurch gekennzeichnet, dass** sie eine Mischung von Rhamnolipiden enthält, wobei in der Mischung das Gewichtsverhältnis von di-Rhamnolipiden zu mono-Rhamnolipiden größer als 51:49, bevorzugt größer 75:25, besonders bevorzugt 97:3, insbesondere größer 98:2 ist.

5. Zusammensetzung gemäß mindestens einem der vorherigen Ansprüche, **dadurch gekennzeichnet, dass** eine wässrige Zusammensetzung ist und einen pH-Wert von 5,5 bis 6,9 bevorzugt von 5,6 bis 6,2, besonders bevorzugt von 5,6 bis 6,0, aufweist.

6. Zusammensetzung gemäß mindestens einem der vorherigen Ansprüche, **dadurch gekennzeichnet, dass** das Rhamnolipid mindestens teilweise als Salz vorliegt und die Kationen der enthaltenen Rhamnolipid-Salze ausgewählt sind aus der Gruppe umfassend, bevorzugt bestehend aus, Li⁺, Na⁺, K⁺, Mg²⁺, Ca²⁺, Al³⁺, NH₄⁺, primäre Ammoniumionen, sekundäre Ammoniumionen, tertiäre Ammoniumionen und quaternäre Ammoniumionen.

7. Zusammensetzung gemäß mindestens einem der vorherigen Ansprüche, **dadurch gekennzeichnet, dass** das Siloxan ausgewählt ist aus der Gruppe umfassend, bevorzugt bestehend aus, Silikonölen, Silikonwachsen, Silicium-funktionellen Siloxanen, organofunktionellen Siloxanen, Polydiethylsiloxanen, Siloxanen aufweisend funktionelle Gruppen und organomodifizierte Siloxanen, wobei organomodifizierte Siloxane, insbesondere Polyethersiloxane bevorzugt sind.

8. Zusammensetzung gemäß mindestens einem der vorherigen Ansprüche, **dadurch gekennzeichnet, dass** das Siloxan in einer Menge von 0,1 ppm bis 5000ppm, bevorzugt von 1 ppm bis 4000 ppm, besonders bevorzugt von 10 ppm bis 2000 ppm, enthalten ist.

9. Formulierung enthaltend mindestens eine Zusammensetzung gemäß mindestens einem der vorherigen Ansprüche.

10. Formulierung gemäß Anspruch 9, **dadurch gekennzeichnet, dass** sie mindestens ein weiteres Tensid enthält.

11. Verwendung mindestens eine Zusammensetzung gemäß mindestens einem der Ansprüche 1 bis 6 oder einer Formulierung gemäß Anspruch 9 oder 10 zur Reinigung von Oberflächen.
